(19) Europäisches Patentamt
European Patent Office
Office européen des brevets

(11) EP 2 482 972 B1

(12) **EUROPEAN PATENT SPECIFICATION**

(45) Date of publication and mention
of the grant of the patent:
**18.12.2019 Bulletin 2019/51**

(21) Application number: **09753503.3**

(22) Date of filing: **30.07.2009**

(51) Int Cl.:
*C07C 68/06* (2006.01)   *C07C 69/96* (2006.01)
*B01J 31/02* (2006.01)   *B01J 27/24* (2006.01)

(86) International application number:
**PCT/CZ2009/000098**

(87) International publication number:
**WO 2009/143785 (03.12.2009 Gazette 2009/49)**

(54) **A PROCESS AND CATALYSTS FOR PRODUCING ALKYLENE AND/OR DIALKYL CARBONATES**

VERFAHREN UND KATALYSATOREN ZUR HERSTELLUNG VON ALKYLEN- UND/ODER
DIALKYLCARBONATEN

PROCÉDÉ ET CATALYSEURS DESTINÉS À LA PRODUCTION DE CARBONATES D'ALKYLÈNE
ET/OU DE DIALKYLE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(43) Date of publication of application:
**08.08.2012 Bulletin 2012/32**

(73) Proprietor: **Majer Laboratory, S.r.o
147 00 Praha 4 (CZ)**

(72) Inventors:
• **DOBRICHOVSKY, Martin
500 11 Hradec Kralove (CZ)**
• **MAJER, Ivan
535 01 Prelouc (CZ)**

(74) Representative: **Jirotkova, Ivana et al
Rott, Ruzicka & Guttmann a spol.
Patent Trademark and Law Offices
Vyskocilova 1566
140 00 Praha 4 (CZ)**

(56) References cited:
**EP-A1- 0 638 541**

Remarks:
The file contains technical information submitted after
the application was filed and not included in this
specification

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

## Description

## Field of the Invention

The present invention relates to a process for the production of dialkyl carbonates, particularly dimethyl and diethyl carbonate, under mild conditions.

## Related Art

Cyclic carbonates with low toxicity, biodegradability and high boiling point are useful solvents and chemical intermediates. They have many applications as inert solvents, processing agents for the production of polyacrylonitrile fibers, diluents for polyurethanes and epoxy resins, accelerant in dyeing and printing, additive in fuel, lube and hydraulic fluids, separation of carbon dioxide and hydrogen sulfide, component of electrolytes in lithium-ion rechargeable batteries, metal extraction, etc. As chemical intermediates, they are used as monomers for the preparation of polycarbonates and other polymeric materials in the field of engineering plastics, precursors for biomedical applications, protecting groups in carbohydrate chemistry.

Cyclic carbonates can be used as a starting material for production of dialkyl carbonates, particularly dimethyl carbonate, via its transesterification with monohydric alcohol, particularly methanol.

Methods of preparing alkylene carbonates are known in the art, however the methods used in the past generally involved rather indirect routes and expensive reactants and often employed reaction mechanisms which were susceptible to steric hindrances.

U.S. Pat. No. 2,773,070, U.S. Pat. No. 4,786,741, U.S. Pat. No. 4,851,555 and U.S. Pat. No. 4,400,559 describe one of the earlier methods of preparing alkylene carbonates which comprises reacting an alkylene oxide with a molar excess of carbon dioxide at a temperature between 90°C and 225°C and a pressure in excess of 2 MPa in the precence of a catalyst comprising one of a specified group of ammonium halides, quarternary phosphonium halides, quarternary arsonium halides and organic sulfonium halides. However, because this industrial process of reacting ethylene oxide with carbon dioxide involves the reaction of explosive ethylene oxide at high pressure, there is a danger of explosion, and various counter-measures against explosion are required in this process.

Early art in the field indicates that cyclic carbonate esters of 1,2-diols can be prepared with thiocyanate salts in the synthesis of episulfides, however the reaction was found to be quite susceptible to steric hindrance. See J. Org. Chem. (1962), 27, 2832.

There is disclosed in U.S. Pat. No. 3,025,305 a process for the production of cyclic carbonates which comprises reacting a monoolefin of about 2 to about 30 carbon atoms with carbon dioxide having a partial pressure of at least about 3.44 MPa and a molecular oxygen-containing gas at a temperature of about 90°C to 200°C and a total pressure sufficient to maintain the liquid phase using two catalysts, a cobalt organic salt and a type of quaternary ammonium compound.

In U.S. Pat. No. 3,923,842 there is disclosed a process for the preparation of an oxirane compound from the corresponding olefin. Here a vicinal halohydrin is formed by reacting the corresponding olefin with oxygen in the presence of an iron halide and a copper halide, under reaction conditions where iron oxide is formed as a coproduct, and is reacted with an amine and carbon dioxide to form one of a group of identified cyclic carbonate esters.

In U.S. Pat. No. 4,009,183 there is disclosed a process for preparing alkylene carbonates from olefins reacted with carbon dioxide in the presence of iodine or certain iodine-containing compounds and an oxygen conveyor at a temperature between 30°C to 120°C and at a pressure between atmospheric to 100 atmospheres and a pH value between 3 and 8.

In U.S. Pat. No. 4,224,223 there is described a process for the preparation of a cyclic alkylene carbonate ester which comprises reacting a cyclic or linear olefin having from 2 to 15 carbon atoms in liquid phase in the presence of oxygen or an oxygen-containing gas and a catalytic amount of an iodine or iodide of a metal and a catalytic iron or copper compound or mixture thereof with carbon dioxide at a temperature of from 50°C to 160°C at a total pressure of from 1.38 MPa to about 13.8 MPa and a pH value of between about 4 and 8.

In DE 3,723,782C (Dainippon Ink Chem KK) cyclo carbonates are prepared from a vicinal halohydrin and alkali bicarbonates by heating in aprotic organic solvents such as dimethyl sulfoxide, acetonitrile and dimethylformamide.

However, these reactions are not eco-friendly or lack economic viability due to risks associated with the use of the poisonous compounds such as phosgene or the low conversion and yield.

Hitherto, various other processes for producing alkylene carbonates have been proposed. Urea can be considered as a potential feed for indirect carbon dioxide utilization. Compared with these traditional routes for synthesis of cyclic carbonates, the route from urea and diols has many advantages, such as cheap and easily available raw material without explosiveness and toxicity, mild reaction conditions, safe operations and higher yield of product.

EP 0,443,758A describes a process of reacting an alkylene glycol and urea at atmospheric pressure or higher either without catalyst or using a tin-containing catalyst. EP 0,443,758A shows an 84 - 99% selectivity of alkylene carbonate to reacted glycol, less than 66% conversion of glycol compared to theoretical conversion and less than 63% selectivity of alkylene carbonate to reacted urea. So a large part of urea either decomposes or not entirely reacts in this process.

As a process of producing a six-membered carbonate, EP 0,057,825 discloses a process for producing a cyclic carbonate from alkylenediol and urea in the presence of an ester-exchange catalyst. This process can not be said to be industrially advantageous, since the yields are as low as about 80% and the reaction time is as long as 10 to 20 hours.

GB 2,280,672 describes a process of reacting internal vicinal diols, particularly 2,3-diols, with urea to form solid carbonates at an elevated temperature and pressure about atmospheric to about 3.5 MPa. It also discloses that using 1,2-diols as a starting material have a disadvantage in formation of ammonium carbamate and oxazolidonones as a degradation and side products, respectively.

U.S Pat. No. 5,349,077 discloses a process when urea reacts with ethylene glycol in a molar ratio in the range 1:2.5 to 1:4 on the dependence of what kind of catalyst is used. The yield is about 79 to 84%. The high ratio of etyhlene glycol and relative low yield of ethylene carbonate has a negative influence on the process economics.

U.S. Pat. No. 5,440,004 discloses the apparatus for performing of reaction of urea with alkylenediols. This special apparatus prevents the deposition of "white crystals" in reflux condenser used in conventional method for producing alkylene carbonates from urea and alkyleneglycols.

U.S. Pat. No. 5,489,702 describes a process of reacting urea with ethylene glycol, where the ethylene glycol is used in the excess in order to increase the selectivity of zinc oxide as a catalyst. Therefore, the presence of ethylene glycol after reaction makes the separation of ethylene carbonate complicated. Moreover, as a preferable way of separating the ethylene glycol from ethylene carbonate is to distill it off. However, ethylene glycol and ethylene carbonate form an azeotropic mixture that makes theirs separation by distillation difficult.

As mentioned above, cyclic carbonates are interesting intermediates for producing dialkylcarbonates by their transesterification with monohydric alcohols, particularly methanol.

Dialkyl carbonates are important commercial compounds, the most important of which is dimethyl carbonate (DMC). Dimethyl carbonate is used as a methylating and carbonylating agent. It can also be used as a solvent to replace halogenated solvents such as chlorobenzene. Although the current price of dimethyl carbonate is prohibitively expensive to use as a fuel additive, it could be used as an oxygenate in reformulated gasoline and an octane component. DMC has a much higher oxygen content (53 wt.%) than MTBE (methyl tertiary butyl ether), TAME (tertiary amyl methyl ether) or ETBE (ethyl tertiary butyl ether) and hence not nearly as much is needed to have the same effect. It has a RON of 130 and is less volatile than either MTBE, TAME or ETBE. It has a pleasant odor and, unlike ethers, is biodegradable. DMC is also replacing phosgene in the production of isocyanates from amines. Under appropriate reaction conditions DMC and an amine react together to form a carbamate. Upon heating the carbamate decomposes to form the desired isocyanate.

In older commercial processes dimethyl carbonate was produced from methanol and phosgene. Because of extreme toxicity and cost of phosgene, there have been efforts to develop better, non-phosgene based processes. For avoiding the use of the phosgene, U.S. Pat. No. 5,162,563, JP 5,616,414,5A and JP 2,193,47A disclosed a method in which methanol is oxidized and carbonylated.

In one new commercial process, dimethyl carbonate is produced from methanol, carbon monoxide, molecular oxygen and cuprous chloride via oxidative carbonylation in a two steps slurry process. Such a process is disclosed in EP 0,460,735A. The major shortcomings of the process are the low production rate, high cost for the separation of products and reactants, formation of by-products, high recycle requirements and the need for corrosion resistant reactors and process lines.

Another new process is disclosed in EP 0,742,198A, EP 0,501,507A, EP 0,538,676A, EP 0,505,374B, JP-A-2-19347 and U.S. Pat. No. 5,162,563 wherein dimethyl carbonate is produced through formation of methyl nitrite instead of the cupric methoxychloride noted above. The by-products are nitrogen oxides, carbon dioxide, methylformate, etc. Dimethyl carbonate in the product stream from the reactor is separated by solvent extractive distillation using dimethyl oxalate as the solvent to break the azeotropic mixture. Although the chemistry looks simple and the production rate is improved, the process is actually very complicated because of the separation of a number of the materials, balancing materials in various flow sections of the process, complicated process control and dealing with the hazardous chemical, methyl nitrite.

U.S. Pat. No. 3,642,858 describes a process in which cyclic carbonate and non-tertiary hydroxy-containing compound are reacted together in the presence of catalytic amount of alkali metal or a derivative thereof to form the carbonate of the hydroxy-containing compound.

U.S. Pat. No. 4,307,032 discloses a process for preparing carbonates of alcohols by contacting a cyclic glycol carbonate with an alcohol at elevated temperature in the presence of thallium compound.

U.S. Pat. No. 4,661,609 discloses the co-synthesis of ethylene glycol and dimethyl carbonate by reacting methanol and ethylene carbonate in the presence of a catalyst selected from zirconium, titanium and tin or compounds or complexes of those metals.

U.S. Pat. No. 4,734,518 discloses the co-synthesis of ethylene glycol and dimethyl carbonate by reacting methanol and ethylene carbonate in the presence of homogenous catalyst. The catalyst is selected from soluble and miscible tertiary phosphines, arsines and stibines, and miscible bivalent sulphur and selenium compounds.

It has been known that dialkyl carbonates can be prepared by reacting primary aliphatic alcohols such as methanol with urea in the presence of various heterogenous and homogenous catalysts such as dibutyltin dimethoxide, tetraphenyltin, etc. See for example P. Ball et al., "Synthesis of Carbonates and Polycarbonates by Reaction of Urea with Hydroxy Compounds", C1 Mol. Chem., 1, pp. 95-108, 1984.

In another commercial process dimethyl carbonate is produced from methanol and carbon dioxide in a two steps process. In the first step cyclic carbonates are produced by reacting epoxides with carbon dioxide or by reacting urea with alkylenediols as described above. In the second step dimethyl carbonate is produced along with alkylenediol by exchange reaction of cyclic carbonate with methanol. See for example Y. Okada, et al. "Dimethyl Carbonate Production for Fuel Additives", ACS, Div. Fuel Chem., Preprint, 41 (3), 868, 1996, John F. Knifton, et al. "Ethylene Glycol-Dimethyl Carbonate Cogeneration", J. Mol. Chem., 67, pp. 389-399, 1991, U.S Pat. No. 5,349,077 and U.S. Pat. No. 5,440,004. As mentioned above, rate of reaction of epoxides with carbon dioxide is slow and requires high pressure, elevated temperature and has safety concerns due to high explosiveness and toxicity of ethylene oxide as a starting material. The second step, the transesterification reaction, is well known and many different catalyst systems have been disclosed. This reaction is generally performed under high pressure and elevated temperature in order to overcome the undesirable equilibria. Even though, the catalytic activity is low, the procedures are complicated and investment costs high.

**Summary of the Invention**

The purpose of this invention is to provide a process for a simple and safe large-scale industrial production of a dialkyl carbonate.

In connection with the deposition of "white crystals", mentioned in U.S. Pat. No. 5,440,004, we have determined that the formation of these crystals depend on the catalyst acidobasic properties. When the catalyst is more acidic, the "white crystals" tend to occur. Also we have been able to determine that the "white crystals" belong to compound ammonium carbamate which is formed when urea decomposes not only to ammonia, but also to carbon dioxide. This decomposition lowers the yields of ethylene carbonate and has a negative influence on economy of the process. In additional to this, we have also found out that if the catalyst - metal oxide - is firstly heated with urea, then urea decomposes to ammonia and water which cause the accurate pH-value 7 or higher in order to form metal cyanate, which is the true catalyst in reaction of urea with alkylene glycols.

According to the present invention, there is provided a process for the production of a dialkyl carbonate by reacting urea and alkylene glycol followed by transesterification of the resulting alkylene carbonate with an alcohol of formula R-OH, wherein R represents an alkyl, in the presence of a metal catalyst, **characterized in that** in the first step urea is reacted with ethylene glycol, in the molar ratio of ethylene glycol to urea being in the range of from 1.10 to 1.00 and in the presence of a catalyst which is a cyanate species of formula $M(CNO)_2$ wherein M stands for the respective metal, obtained by an *in situ* reaction of urea and the metal oxide, the catalyst being prepared *in situ* by fusing the metal oxide and urea under atmospheric pressure and a temperature around 135 °C, and where the metal is selected from zinc and magnesium, until dissolution of the metal oxide forming a pellucid solution prior to the addition of ethylene glycol, to form said alkylene carbonate, whereupon in the second step of transesterification, 10 moles of the monohydric alcohol is used per 1 mole of the alkylene carbonate, the transesterification is performed at reflux temperature and atmospheric pressure, the transesterification being performed directly from the first step and with the same catalyst as in the first step

Accordingly, the process of the present invention is carried out two-steps in one, where ethylene carbonate carbonate is prepared by reaction of urea and ethylene glycol in the presence of a catalyst (first step) and said ethylene carbonate is transesterficated in the presence of a catalyst (second step) by a monohydric alcohol to form dialkyl carbonate and ethylene glycol which is recyclable.

From the available art it does not appear that any one skilled in the art have heretofore considered a method for preparing dialkyl carbonates, preferably dimethyl carbonate and diethyl carbonate, from the corresponding alcohol by transesterification of alkylene carbonate, i.e. ethylene carbonate where the catalyst for the first step is a reaction product of urea and metal compound, preferably metal oxide and the catalyst for the second step is prepared by the same way as in the first step. If the catalysts are prepared by this way the first reaction is almost quantitative in nearly equimolar ration of urea and alkylene glycols and the second reaction is perfomed in excellent yields with nearly 100% selectivities. In additional to this, since the catalyst contains cyanate species, it can be prepared by other known methods, e.g. by reaction of metal carbonates and hydroxycarbonates with urea, or other nitrogen-containing compounds such as e.g. carbamates.

**Detailed Description of the Invention**

The present invention will be described in more detail below, referring to Examples, which are not intended to limit the scope of the present invention.

The inventors have been engaged in intensive research to attain the above stated purpose. The process comprises reaction of urea with ethylene glycol in order to form corresponding alkylene carbonate and the transesterification reaction of said alkylene carbonate with monohydric alcohol.

The preparation of the catalyst is extremely simple and straightforward and is realized by fusing a mixture of the metal compound and urea *in situ* in the vessel under atmospheric pressure and/or slight overpressure at a temperature around 135°C according to the following reaction scheme, if the metal compound is the metal oxide:

$$MO + 2\,urea = M(CNO)_2 + 2\,NH_3 + H_2O$$

wherein M stands for the respective metal.

To ensure for the first step a successful accomplishment without side-products and by-products, it is sufficient that only 20% of the used metal compound are changed into metal cyanate. To achieve this goal 1% surplus of urea is sufficient.

Although it is not necessary the catalyst can be comprised from different metals. The catalyst can be used as a catalyst by themselves or with inert compounds, or with carriers supporting the catalyst.

For the first step, the molar ratio of the ethylene glycol to urea is in the range of 0.90 to 1.10, preferably 1.00 to 1.10, more preferably 1.06. The slight excess of glycol is due to its high vapour pressure not due to catalyst's low selectivity.

A solvent is not necessary to perform the first reaction of the present invention because the reaction is performed under slight excess of glycol due to its high vapour pressure. In additional to this, as another object of this invention, the first step can be performed in the presence of alkylene carbonate as a solvent. Therefore, this reaction can be easily performed as a continuous process. However, a solvent that is inert under the reaction conditions may be used.

The amount of the catalyst used in the first step is not limited, however, the molar ratio of metal to urea is generally within the range of 0.0001 to 10, and the preferable range is from 0.001 to 1.0.

The first step of the present invention is performed by maintaining the reaction mixture of urea, ethylene glycol and catalyst at constant external and/or internal temperature and reduced pressure determined by Antoin equation, simultaneously removing ammonia which is produced from the mixture as a by-product of the first reaction.

The ammonia may be removed by introducing inert gas to the reacted solution under the reaction conditions.

Generally the temperature range for the first reaction is from about 120°C to 200°C. The reaction rate is small at temperature lower than 120°C, and the amount of side products is increased at temperature higher than 200°C.

The reaction time depends on the kind of raw glycols, the molar ratio of glycol to urea, the type and amount of catalyst, the reaction temperature and reaction pressure etc... The prefered residence time is in the range of 1 to 10 hours.

The reaction pressure depends on the reaction temperature and the composition of the reaction solution. The reaction can be performed under atmospheric pressure or higher, however, the reaction is preferably performed under reduced pressures of 7.5 to 600 mmHg absolute. Higher selectivity is obtained at reduced pressure when reaction temperature is lower.

According to this invention e.g. zinc oxide and urea are introduced into the reaction vessel under atmospheric pressure and heated up until the zinc oxide dissolves and pellucid solution is formed. Part of zinc oxide turns into the zinc cyanate in order to form the catalyst and ammonia is released. Then, into this, preferably cooled mixture ethylene glycol is added. The reaction mixture is heated up to the desired temperature and reduced pressured and afterwards ammonia is released. The alkylene carbonate produced is directly introduced to the second step of this invention.

It has been found that reaction product of reaction urea with metal compound, zinc or magnesium oxide, are excellent catalysts for the transesterification reaction of alkylene carbonates according to this invention, ethylene carbonate with monohydric alcohols, preferably with methanol and ethanol in order to form corresponding dialkyl carbonates and vicinal diols.

The transesterification may be performed as a second step directly from the first step of preparing the alkylene carbonate by adding the desired alcohol and heating up the reaction mixture to the reflux temperature.

The molar ratio of the alkylene carbonate to alcohol is 1:10.

Although it is not necessary, this reaction can be performed in the presence of high boiling point solvent. Examples of preferable high boiling point solvent include hydrocarbons and ethers. Although the hydrocarbons may be aliphatic unsaturated hydrocarbons, saturated hydrocarbons or aromatic hydrocarbons having high stability are preferable. Ethers may be aromatic ethers, aliphatic ethers or aromatic aliphatic ethers.

Examples of preferable hydrocarbons solvent include undecane, dodecane, tridecane, tetradecane, pentadecane, hexadecane, heptadecane, octadecane, nonadecane, eicosane, tetramethylpentadecane, dicyclohexyl, hexylbenzene, cyclohexylbenzene, heptylbenzene, octylbenzene, nonylbenzene, decylbenzene, undecylbenzene, diisopropylbenzene, triisopropylbenzene, pentamethylbenzene, methylnaphthalene, diphenylmethane, ethylbiphenyl, bibenzyl and isomers thereof.

Examples of preferable ether solvent include dihexyl ether, dioctyl ether, cyclododecyl methyl ether, diethyleneglycol dimethyl ether, diethyleneglycol dibutyl ether, triethyleneglycol dimethyl ether, tetraethyleneglycol dimethyl ether, butyl phenyl ether, dibenzyl ether, diphenyl ether, ditolyl ether and isomers thereof.

The amount of the catalyst used is not limited, however, the molar ratio of metal and urea to alkylene carbonate is generally within the range of 0.0001 to 10, and the preferable range is from 0.001 to 2.0.

The transesterification of the present invention is performed by maintaining the reaction mixture of ethylene glycol carbonate, alcohol and catalyst at reflux temperature and atmospheric pressure. Generally the reflux temperature range is from about 62°C to 70°C. Although it is not necessary the second step of this invention can be performed under an inert atmosphere.

The reaction time depends on the kind of raw carbonates and alcohols, the molar ratio of carbonates to alcohols, the type and amount of catalyst, the reaction temperature and reflux ratio of alcohol-dialkyl carbonate azeotrope. The preferred residence time is in the range of 1 to 20 hours.

According to this invention zinc/magnesium oxide and urea are introduced into the reaction vessel under atmospheric pressure and heated up until the zinc/magnesium oxide dissolve and pellucid solution is formed. Part of zinc/magnesium oxide turn into the zinc/magnesium cyanate in order to form the catalyst and ammonia is released. Then, into this, preferably cooled mixture is added solution of ethylene carbonate in desired alcohol. The reaction mixture is heated up to the reflux temperature under atmospheric pressure. The dialkyl carbonates can be separated from the reaction mixture by simple distillation of the azeotropic mixture. The azeotrope can be broken either by extractive distillation as was described, for example, in U.S. Pat. No. 4,162,200 or, for instance, by pressure distillation as was described, for example, in DE 2,607,003.

After distillation, the residue which contains mainly vicinal diol, catalyst and small part of unreacted ethylene carbonate can be directly without further purification transferred to the first step of this invention or, more preferably, filtered the catalyst off the residue, which is recycled to the first step and the catalyst is recycled to the second step. However, if it is necessary, the purification process can be included.

For recycling the catalyst used and recovered in the second step to the first step, it is preferred to use the same catalyst in the first and second steps.

The process of instant invention may be a batch or a continuous process. The rate of reaction and related economics determine which type of process is preferred. However, both steps preferably run as a continuous process. The first step of the process, for example, as a serial Continuous-Flow Stirred Tank Reactors (CSTR's), the second step of the process runs continuously with a simultaneously separation of distillation residue and the carbonates products. Distillation residue is preferably introduced after filtering the catalyst off to the first reaction without further purification.

In a batch process of the second step, it is optionally that total amount of alcohol is not added at the starting time of reaction, but gradually added with progress of the reaction.

Products have been identified in this work by gas chromatography (GC); all temperatures are in degrees centigrade and all pressures are in mmHg absolute.

## Example 1

As a first step of this invention there was charged into a 500-mL three-necked flask equipped with a magnetic stirrer 61.0 g (1.02 mol) of urea and 3.2 g zinc oxide. The mixture was heated up until urea melted, zinc oxide dissolved into a pellucid solution and ammonia started to release. After about 2 to 5 minutes when the weight of the mixture decreased about 1.0 gram, the catalyst as a product of reaction of urea and zinc oxide was formed. Afterwards, into this cooled solidified mixture of urea and catalyst, 65.8 g (1.06 mol) of ethylene glycol was charged. The flask was then equipped with a thermometer, condenser and the pressure was reduced to 22.5 mmHg and the mixture was heated to internal temperature 138°C for 3 hours.

Then the reaction mixture was cooled; the amount of the reaction mixture was 94.9 g. The reaction mixture was analyzed for a composition by gas chromatography to show 87.9 g of formed ethylene carbonate and 3.8 g of unreacted ethylene glycol.

Therefore, the conversion of ethylene glycol is 94.2% (theoretical conversion is 94.2%), the selectivity of ethylene carbonate on the basis of the reacted ethylene glycol is 99.8%, and the selectivity to ethylene carbonate on the basis of urea is 99.8%. The conversion of urea is 100%.

As a second step of this invention there was charged into the reaction mixture after the first step 320.0 g (10.0 mol) of methanol. The mixture was heated up to reflux temperature about 65°C for 5 hours under atmospheric pressure.

Then the reaction was cooled; the amount of the reaction mixture was 414.9 g. The reaction mixture was analyzed for a composition by gas chromatography to show 8.8 g of unreacted ethylene carbonate, 59.5 g of ethylene glycol, 80.3 g of dimethyl carbonate and 262.5 g of methanol.

Therefore, the conversion of ethylene carbonate is 90.0%, and the selectivity to dimethyl carbonate on the basis of reacted ethylene carbonate is 99.3%.

## Reference Example 2

A reaction mixture was obtained in the same manner as in the first step of Example 1.

As a second step of this invention there was charged into 1-L flask the entire amount of the reaction mixture obtained in the first step and 460.1 g (10.0 mol) of ethanol. The mixture was heated up to reflux temperature about 77°C for 5 hours under atmospheric pressure.

Then the reaction mixture was cooled; the amount of the reaction mixture was 555.0 g. The reaction mixture was analyzed for a composition by gas chromatography to show 22.9 g of unreacted ethylene carbonate, 40.4 g of ethylene glycol, 76.9 g of diethyl carbonate.

Therefore, the conversion of ethylene carbonate is 73.9%, and the selectivity to diethyl carbonate on the basis of reacted ethylene carbonate is 88.0%.

**Reference Example 3**

As a second step of this invention performed directly with pure ethylene carbonate there was charged into 50-mL flask 0.3 g of zinc oxide and 0.3 g of urea. The mixture was heated up for about 5 minutes until the pellucid solution formed. Then, solution of 8.8 g ethylene carbonate in 32.0 g of methanol was added and the reaction mixture was heated up to reflux temperature for 5 hours under atmospheric pressure.

Then the reaction was cooled; the amount of the reaction mixture was 41.3 g. The reaction mixture was analyzed for a composition by gas chromatography to show 0.8 g of unreacted ethylene carbonate, 5.6 g of ethylene glycol, 8.1 g of dimethyl carbonate and 26.1 g of methanol.

Therefore, the conversion of ethylene carbonate is 90.9%, and the selectivity to dimethyl carbonate on the basis of reacted ethylene carbonate is 99.0%.

**Reference Example 4**

The Example 3 was repeated except that the zinc oxide was replaced with 0.3 g of magnesium oxide.

The amount of the reaction mixture after reaction was 41.3 g. The reaction mixture was analyzed for a composition by gas chromatography to show 0.8 g of unreacted ethylene carbonate, 5.4 g of ethylene glycol, 7.8 g of dimethyl carbonate and 26.4 g of methanol.

Therefore, the conversion of ethylene carbonate is 90.9%, and the selectivity to dimethyl carbonate on the basis of reacted ethylene carbonate is 95.3%.

**Reference Example 5**

The Example 3 was repeated except that the methanol was replaced with 60.0 g of propylalcohol.

The amount of the reaction mixture after reaction was 69.3 g. The reaction mixture was analyzed for a composition by gas chromatography to show 2.4 g of unreacted ethylene carbonate, 4.4 g of ethylene glycol and 10.3 g of dipropyl carbonate.

Therefore, the conversion of ethylene carbonate is 72.7%, and the selectivity to dipropyl carbonate on the basis of reacted ethylene carbonate is 96.9%.

Reference example 6

The Example 1 was repeated except that the ethylene glycol was replaced with 80.7 g of propylene glycol.

The amount of the reaction mixture after the first step was 109.8 g. The reaction mixture was analyzed for a composition by gas chromatography to show 102.0 g of formed propylene carbonate and 4.6 g of unreacted propylene glycol.

Therefore, the conversion of propylene glycol is 94.3% (theoretical conversion is 94.3%), the selectivity of propylene carbonate on the basis of the reacted propylene glycol is 99.1%, and the selectivity to propylene carbonate on the basis of urea is 99.1%. The conversion of urea is 100%.

As a second step of this invention there was charged into the reaction mixture after the first step 320.0 g (10.0 mol) of methanol. The mixture was heated up to reflux temperature about 65°C for 5 hours under atmospheric pressure.

Then the reaction was cooled; the amount of the reaction mixture was 429.8 g. The reaction mixture was analyzed for a composition by gas chromatography to show 41.8 g of unreacted propylene carbonate, 44.8 g of propylene glycol and 53.0 g of dimethyl carbonate.

Therefore, the conversion of propylene carbonate is 59.0%, and the selectivity to dimethyl carbonate on the basis of reacted propylene carbonate is 99.8%.

**Reference Example 7**

The Example 3 was repeated except that the ethylene carbonate and methanol were replaced with 10.2 g of propylene carbonate and 46.1 g of ethanol.

The amount of the reaction mixture after reaction was 56.8 g. The reaction mixture was analyzed for a composition by gas chromatography to show 6.2 g of unreacted propylene carbonate, 2.3 g of propylene glycol and 3.6 g of diethyl carbonate.

Therefore, the conversion of propylene carbonate is 39.2%, and the selectivity to diethyl carbonate on the basis of reacted propylene carbonate is 77.8%.

**Example 8**

The first step of the Example 1 was repeated except that zinc oxide was replaced with 6.8 g of $Zn(CNO)_2$ and the amount of urea was 60.0 g.

The amount of the reaction mixture after reaction was 97.7 g. The reaction mixture was analyzed for a composition by gas chromatography to show 86.2 g of formed ethylene carbonate and 3.8 g of unreacted ethylene glycol.

Therefore, the conversion of ethylene glycol is 94.2% (theoretical conversion is 94.2%), the selectivity of ethylene carbonate on the basis of the reacted ethylene glycol is 97.9%, and the selectivity to ethylene carbonate on the basis of urea is 97.9%. The conversion of urea is 100%.

**Reference Example 9**

The Example 3 was repeated except that the zinc oxide was replaced with 0.5 g of zinc cyanate.

The amount of the reaction mixture after reaction was 41.3 g. The reaction mixture was analyzed for a composition by gas chromatography to show 0.8 g of unreacted ethylene carbonate, 5.6 g of ethylene glycol and 8.2 g of dimethyl carbonate.

Therefore, the conversion of ethylene carbonate is 90.9%, and the selectivity to dimethyl carbonate on the basis of reacted ethylene carbonate is 99.8%.

**Reference Example 10**

The Example 3 was repeated except that the zinc oxide was replaced with 0.3 g of calcium oxide.

The amount of the reaction mixture after reaction was 41.3 g. The reaction mixture was analyzed for a composition by gas chromatography to show 0.8 g of unreacted ethylene carbonate, 5.5 g of ethylene glycol and 8.0 g of dimethyl carbonate.

Therefore, the conversion of ethylene carbonate is 90.9%, and the selectivity to dimethyl carbonate on the basis of reacted ethylene carbonate is 97.8%.

Example 11

The first step of the Example 1 was repeated except that 88.0 g of ethylene carbonate was added with 65.8 g of ethylene glycol.

The amount of the reaction mixture after reaction was 181.8 g. The reaction mixture was analyzed for a composition by gas chromatography to show 84.3 g of formed ethylene carbonate and 4.2 g of unreacted ethylene glycol.

Therefore, the conversion of ethylene glycol is 93.2% (theoretical conversion is 94.2%), the selectivity of ethylene carbonate on the basis of the reacted ethylene glycol is 96.4%, and the selectivity to ethylene carbonate on the basis of urea is 95.8%. The conversion of urea is 100%.

**Example 12**

The first step of the Example 1 was repeated except that the reaction was performed at constant external temperature 165°C instead of constant internal temperature.

The amount of the reaction mixture after reaction was 94.9 g. The reaction mixture was analyzed for a composition by gas chromatography to show 87.3 g of formed ethylene carbonate and 3.7 g of unreacted ethylene glycol.

Therefore, the conversion of ethylene glycol is 94.0% (theoretical conversion is 94.2%), the selectivity of ethylene carbonate on the basis of the reacted ethylene glycol is 99.0%, and the selectivity to ethylene carbonate on the basis of urea is 99.2%. The conversion of urea is 100%.

**Example 13**

The first step of the Example 1 was repeated except that the zinc oxide was replaced with 4.9 g of zinc carbonate.

The amount of the reaction mixture after reaction was 96.2 g. The reaction mixture was analyzed for a composition by gas chromatography to show 86.8 g of formed ethylene carbonate and 3.8 g of unreacted ethylene glycol.

Therefore, the conversion of ethylene glycol is 94.2% (theoretical conversion is 94.2%), the selectivity of ethylene carbonate on the basis of the reacted ethylene glycol is 97.9%, and the selectivity to ethylene carbonate on the basis of urea is 97.9%. The conversion of urea is 100%.

**Reference Example 14**

The Example 3 was repeated except that the urea was replaced with 0.5 g of 2-hydroxyethyl carbamate as a nitrogen-containing compound.

The amount of the reaction mixture after reaction was 41.5 g. The reaction mixture was analyzed for a composition by gas chromatography to show 3.2 g of unreacted ethylene carbonate, 2.7 g of ethylene glycol and 3.9 g of dimethyl carbonate.

Therefore, the conversion of ethylene carbonate is 63.6%, and the selectivity to dimethyl carbonate on the basis of reacted ethylene carbonate is 68.1%.

**Comparative Example 1**

The Example 3 was repeated except that urea was not added.

The amount of the reaction mixture was 41.1 g. The reaction mixture was analyzed for a composition by gas chromatography to show 6.2 g of unreacted ethylene carbonate, 0.3 g of ethylene glycol and 0.5 g of dimethyl carbonate.

Therefore, the conversion of ethylene carbonate is 29.5%, and the selectivity to dimethyl carbonate on the basis of reacted ethylene carbonate is 18.8%.

**Comparative Example 2**

The Example 4 was repeated except that urea was not added.

The amount of the reaction mixture was 41.1 g. The reaction mixture was analyzed for a composition by gas chromatography to show 6.3 g of unreacted ethylene carbonate, 0.3 g of ethylene glycol and 0.4 g of dimethyl carbonate.

Therefore, the conversion of ethylene carbonate is 28.4%, and the selectivity to dimethyl carbonate on the basis of reacted ethylene carbonate is 15.6%.

**Comparative Example 3**

The first step of the Example 1 was repeated except that 3.2 g of zinc oxide, 60.0 g of urea and 65.8 g of ethylene glycol were added together without preparation of composite catalyst.
The amount of the reaction mixture after reaction was 93.1 g. The reaction mixture was analyzed for a composition by gas chromatography to show 62.3 g of formed ethylene carbonate and 13.4 g of unreacted ethylene glycol.
Therefore, the conversion of ethylene glycol is 79.6% (theoretical conversion is 94.2%), the selectivity of ethylene carbonate on the basis of the reacted ethylene glycol is 83.7%, and the selectivity to ethylene carbonate on the basis of urea is 70.8%. The conversion of urea is 100%.

**Claims**

1. A process for the production of a dialkyl carbonate by reacting urea and alkylene glycol followed by transesterification of the resulting alkylene carbonate with an alcohol of formula R-OH, wherein R represents an alkyl, in the presence of a metal catalyst, **characterized in that** in the first step urea is reacted with ethylene glycol, in the molar ratio of ethylene glycol to urea being in the range of from 1.10 to 1.00 and in the presence of a catalyst which is a cyanate species of formula $M(CNO)_2$ wherein M stands for the respective metal, obtained by an *in situ* reaction of urea and the metal oxide, the catalyst being prepared *in situ* by fusing the metal oxide and urea under atmospheric pressure and a temperature around 135 °C, and where the metal is selected from zinc and magnesium, until dissolution of the metal oxide forming a pellucid solution prior to the addition of ethylene glycol, to form said alkylene carbonate, whereupon in the second step of transesterification, 10 moles of the monohydric alcohol is used per 1 mole of the alkylene carbonate, the transesterification is performed at reflux temperature and atmospheric pressure, the transesterification being performed directly from the first step and with the same catalyst as in the first step.

2. A process as claimed in claim 1, wherein the metal is zinc.

3. A process as claimed in claim 2, wherein the process comprises further steps in which:

   (a) the formed dialkyl carbonate is separated from ethylene glycol, and optionally
   (b) ethylene glycol is recycled to the first step of claim 1.

4. A process as claimed in any one of the claims 1 to 3, wherein the said alcohol contains from 1 to 16 carbon atoms.

5. A process as claimed in any one of claims 2 to 4, wherein the catalyst present in the product mixture from the transesterification is recycled either to the preparation of the alkylene carbonate or to the transesterification.

**Patentansprüche**

1. Verfahren zur Herstellung eines Dialkylcarbonats durch Umsetzung von Harnstoff und Alkylenglykol und anschließende Umesterung des entstandenen Alkylencarbonats mit einem Alkohol der Formel R-OH, worin R für ein Alkyl steht, in Gegenwart eines Metallkatalysators, **dadurch gekennzeichnet, dass** im ersten Schritt Harnstoff mit Ethylenglykol umgesetzt wird, wobei das Molverhältnis von Ethylenglykol zu Harnstoff im Bereich von 1,10 bis 1,00 liegt und in Gegenwart eines Katalysators, der eine Cyanatspezies der Formel $M(CNO)_2$ ist, worin M steht für das jeweilige Metall, das durch eine in situ-Reaktion von Harnstoff und Metalloxid erhalten wird, wobei der Katalysator in situ durch Schmelzen des Metalloxids und des Harnstoffs unter Atmosphärendruck und einer Temperatur um 135°C hergestellt wird und wobei das Metall ausgewählt wird von Zink und Magnesium, bis zur Auflösung des Metalloxids unter Bildung einer pelluziden Lösung vor der Zugabe von Ethylenglykol, um das Alkylencarbonat zu bilden, woraufhin im zweiten Umesterungsschritt 10 Mol des einwertigen Alkohols wird pro 1 Mol des Alkylencarbonats eingesetzt, die Umesterung wird bei Rückflußtemperatur und Normaldruck durchgeführt, wobei die Umesterung direkt aus der ersten Stufe und mit dem gleichen Katalysator wie in der ersten Stufe durchgeführt wird.

2. Verfahren nach Anspruch 1, **dadurch gekennzeichnet, dass** das Metall Zink ist.

3. Verfahren nach Anspruch 2, wobei das Verfahren weitere Schritte umfasst, in denen:

   (a) das gebildete Dialkylcarbonat wird von Ethylenglykol abgetrennt. und gegebenenfalls
   (b) Ethylenglykol wird in die erste Stufe von Anspruch 1 zurückgeführt.

4. Verfahren nach einem der Ansprüche 1 bis 3, wobei der Alkohol 1 bis 16 Kohlenstoffatome enthält.

5. Verfahren nach einem der Ansprüche 2 bis 4, **da-**

**durch gekennzeichnet, dass** der in der Produktmischung aus der Umesterung enthaltene Katalysator entweder zur Herstellung des Alkylencarbonats oder zur Umesterung zurückgeführt wird.

## Revendications

1. Procédé de préparation d'un carbonate de dialkyle par réaction d'urée et d'alkylène glycol, suivi d'une transestérification du carbonate d'alkylène résultant avec un alcool de formule R-OH, dans laquelle R représente un groupe alkyle, en présence d'un catalyseur métallique, *caractérisé en ce que* dans la première étape, on fait réagir de l'urée avec de l'éthylène glycol, le rapport molaire de l'éthylène glycol à l'urée étant compris entre 1,10 et 1,00, et en présence d'un catalyseur qui est une espèce cyanate de formule $M(CNO)_2$, M représente le métal respectif, obtenu par une réaction in situ de l'urée et de l'oxyde métallique, le catalyseur étant préparé in situ par fusion de l'oxyde métallique et de l'urée sous pression atmosphérique et à une température d'environ 135°C, et dans lequel le métal est sélectionné parmi le zinc et le magnésium, jusqu'à la dissolution de l'oxyde métallique formant une solution pellucide avant l'addition d'éthylène glycol, pour former ledit carbonate d'alkylène, après quoi dans la deuxième étape de transestérification, 10 moles d'alcool monohydrique par mole de carbonate d'alkylène, la transestérification est effectuée à la température de reflux et à la pression atmosphérique, la transestérification étant réalisée directement à partir de la première étape et avec le même catalyseur qu'à la première étape.

2. Procédé selon la revendication 1, **caractérisé en ce que** le métal est le zinc.

3. Procédé selon la revendication 2, **caractérisé en ce qu'**il comprend d'autres étapes dans lesquelles:

   (a) le carbonate de dialkyle formé est séparé de l'éthylène glycol, et éventuellement
   (b) l'éthylène glycol est recyclé à la première étape de la revendication 1.

4. Procédé selon l'une quelconque des revendications 1 à 3, dans lequel ledit alcool contient de 1 à 16 atomes de carbone.

5. Procédé selon l'une quelconque des revendications 2 à 4, dans lequel le catalyseur présent dans le mélange de produits provenant de la transestérification est recyclé soit dans la préparation du carbonate d'alkylène, soit dans la transestérification.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2773070 A
- US 4786741 A
- US 4851555 A
- US 4400559 A
- US 3025305 A
- US 3923842 A
- US 4009183 A
- US 4224223 A
- DE 3723782 C
- EP 0443758 A
- EP 0057825 A
- GB 2280672 A
- US 5349077 A
- US 5440004 A
- US 5489702 A
- US 5162563 A
- JP 56164145 A
- JP 219347 A
- EP 0460735 A
- EP 0742198 A
- EP 0501507 A
- EP 0538676 A
- EP 0505374 B
- JP 2019347 A
- US 3642858 A
- US 4307032 A
- US 4661609 A
- US 4734518 A
- US 4162200 A
- DE 2607003

**Non-patent literature cited in the description**

- *J. Org. Chem.,* 1962, vol. 27, 2832
- **P. BALL et al.** Synthesis of Carbonates and Polycarbonates by Reaction of Urea with Hydroxy Compounds. *C1 Mol. Chem.,* 1984, vol. 1, 95-108
- **Y. OKADA et al.** Dimethyl Carbonate Production for Fuel Additives. *ACS, Div. Fuel Chem.,* 1996, vol. 41 (3), 868
- **JOHN F. KNIFTON et al.** Ethylene Glycol-Dimethyl Carbonate Cogeneration. *J. Mol. Chem.,* 1991, vol. 67, 389-399